# EUROPEAN PATENT SPECIFICATION

(11) **EP 4 343 987 B1**
(45) Date of publication and mention of the grant of the patent: **15.07.2026**
(21) Application number: 22946045.6
(22) Date of filing: 29.06.2022
(51) Int. Cl.: H01R 39/08, H01R 39/64, A61B 6/00, A61N 5/10

(54) **SLIP RING ASSEMBLY AND MEDICAL DEVICE HAVING SAME**
GLEITRINGANORDNUNG UND MEDIZINISCHE VORRICHTUNG DAMIT
ENSEMBLE BAGUE COLLECTRICE ET DISPOSITIF MÉDICAL LE COMPRENANT

(43) Date of publication of application: 27.03.2024
(73) Proprietor: Shanghai United Imaging Healthcare Co., Ltd., Shanghai, 201807 (CN)
(72) Inventor: ZOU, Jianxiong, Shanghai 201807 (CN); FU, Feichao, Shanghai 201807 (CN); WANG, Peng, Shanghai 201807 (CN); NI, Cheng, Shanghai 201807 (CN); XING, Tuota, Shanghai 201807 (CN)
(74) Representative: Murgitroyd & Company
(86) International application number: PCT/CN2022/102544
(87) International publication number: WO 2024/000326

(56) References cited:
- EP-B1- 2 648 613
- CN-A- 1 915 173
- CN-A- 103 260 511
- CN-A- 103 282 081
- CN-A- 112 349 670
- CN-U- 215 732 606
- GB-A- 780 146
- US-A- 5 734 218

## Description

### TECHNICAL FIELD

The present application relates to the field of magnetic resonance medical technology, and in particular to a slip ring assembly, and a medical device having the slip ring assembly.

### BACKGROUND

In modem medicine, imaging equipment is usually used to obtain images of patients to make it easy for a doctor to diagnose or to treat patients by using treatment equipment. For example, magnetic resonance imaging devices are used to obtain magnetic resonance images of tissues of patients, such as tumor patients, to clearly display the conditions of the diseased site and surrounding tissues, so that the diseased tissue can be accurately located. Further, a precise radiation therapy is performed by using the treatment equipment (such as an electronic linear accelerator) based on magnetic resonance images. Magnetic resonance and the treatment device may be used in combination, and the slip ring assembly may be used as a power supply medium and a communication medium of the treatment device. When the slip ring assembly is powered on, the magnetic field generated by the current flowing through the slip ring assembly will interfere with the central magnetic field of the MRI device, thus resulting in a lower accuracy of MRI. Document EP2648613B1 discloses a medical apparatus comprising a magnetic resonance imaging system for acquiring magnetic resonance data.

### SUMMARY

Based on this, in order to address the above technical problems, it is necessary to provide a slip ring assembly and a medical device having the slip ring assembly, which can effectively reduce the interference of a magnetic field generated by a current flowing through the slip ring assembly in the central magnetic field of the MRI device, and which has a compact structure and requires a relatively small installation space. The invention is set out in the appended set of claims.

### BRIEF DESCRIPTION OF THE DRAWINGS

The above and other objectives, features and advantages of the present invention will become more apparent through a more particular description of the preferred embodiments of the present invention as shown in the accompanying drawings. The same reference numerals refer to the same parts throughout the drawings. The drawings are not intentionally drawn to scale to actual size, but intended to emphasize and illustrate the subject of the present invention.

Other features, objectives and advantages of the present invention will become more apparent upon reading the detailed description of the non-limiting embodiments with reference to the following drawings:
FIG. 1 is a perspective diagram of a medical device according to an embodiment not forming part of the present application;
FIG. 2 is a schematic plan view of a slip ring assembly in the prior art;
FIG. 3 is a schematic plan view of a slip ring assembly according to an embodiment not forming part of the present application;
FIG. 4 is a perspective diagram of the slip ring assembly according to an embodiment not forming part of the present application;
FIG. 5 is a schematic sectional view of the slip ring assembly shown in FIG. 4;
FIG. 6 is a perspective diagram of the slip ring assembly according to an embodiment not forming part of the present application;
FIG. 7 is a schematic sectional view of the slip ring assembly shown in FIG. 6;
FIG. 8 is a schematic sectional view of the slip ring assembly according to an embodiment not forming part of the present application;
FIG. 9 is a perspective diagram of the slip ring assembly according to an embodiment of the present application;
FIG. 10 is a schematic sectional view of the slip ring assembly shown in FIG. 9;
FIG. 11 is a perspective diagram of the slip ring assembly according to an embodiment of the present application;
FIG. 12 is a schematic sectional view of the slip ring assembly shown in FIG. 11;
FIG. 13 is a perspective diagram of the slip ring assembly according to an embodiment of the present application;
FIG. 14 is a schematic sectional view of the slip ring assembly shown in FIG. 13;
FIG. 15 is a schematic sectional view of the slip ring assembly according to an embodiment of the present application;
FIG. 16 is a schematic sectional view of the slip ring assembly according to an embodiment of the present application; and
FIG. 17 is a schematic sectional view of the slip ring assembly according to an embodiment of the present application.

### DETAILED DESCRIPTION OF THE EMBODIMENTS

In order to facilitate understanding of the present application, the present application will be described more completely hereinafter with reference to the relevant drawings. Preferred embodiments of the present application are shown in the accompanying drawings, however, the present application may be implemented in many different forms and is not limited to the embodiments described herein. Rather, these embodiments are provided so as to make the disclosure of the present application more thorough and complete.

It should be noted that, when an element is referred to as "connected" to another element, it may be directly connected to and integral with the other element, or there may be intervening elements therebetween. The terms "installation", "one end", "another end", and similar expressions used herein are for illustrative purposes only.

Unless otherwise defined, all technical and scientific terms used herein have the same meaning as commonly understood by one of the ordinary skilled in the technical field of this application. The terminology used herein in the description of the application is for the purpose of describing specific embodiments only and is not intended to limit the application. The term "and/or" herein includes any and all combinations of one or more of the associated listed items.

In order to make the purpose, technical solutions and advantages of the present application clearer, the present application will be further described in detail hereinafter with reference to the drawings and embodiments. It should be understood that the specific embodiments described herein are only used to explain the present application, but not intended to limit the present application.

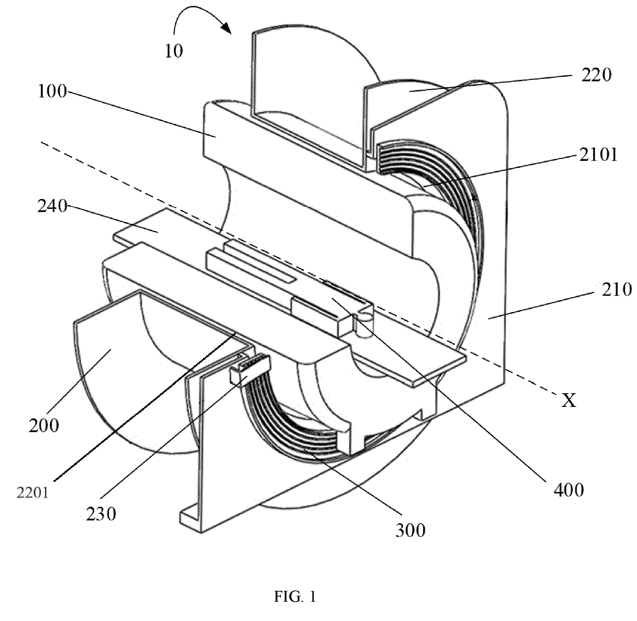

Referring to FIG. 1, FIG. 1 is a perspective diagram of a medical device 10 according to an embodiment of the present application. The medical device 10 includes: a magnetic resonance imaging (MRI) device 100, a treatment device 200, and a bed assembly 240. The MRI device 100 is configured to be in a cylindrical shape and have a hollow portion. The treatment device 200 is, for example, a linear accelerator 200. Optionally, the treatment device 200 may also be any other suitable treatment device, which is not limited in this application. The linear accelerator 200 includes: an accelerator stator 210, an accelerator roller 220, and an electrically connecting terminal 230. The accelerator stator 210 is fixedly arranged, and the accelerator stator 210 has a circular first opening 2101. The accelerator roller 220 has a circular second opening 2201, and the second opening 2201 and the first opening 2101 have a common axis X. The accelerator roller 220 is configured to be pivotable around the axis X relative to the accelerator stator 210. The slip ring assembly 300 is provided on one side of the accelerator roller 220, and the slip ring assembly 300 is arranged coaxially with the first opening 2101 and the second opening 2201. The electrically connecting terminal 230 is disposed on the accelerator stator 210 and may be electrically connected to the slip ring assembly 300. In this embodiment, the electrically connecting terminal 230 is, for example, a carbon brush 230. Optionally, the electrically connecting terminal 230 may also be any other suitable electrical connection device, such as a metal brush, etc., which is not limited in the application, as long as it may be ensured that the slip ring assembly 300 is electrically connected to the linear accelerator 200. In this embodiment, the slip ring assembly 300 is arranged on the accelerator roller 220, and the electrically connecting terminal 230 is arranged on the accelerator stator 210. When the linear accelerator 200 operates, the slip ring assembly 300 pivots together with the accelerator roller 220, and the slip ring assembly 300 is electrically connected to the linear accelerator 200 by the carbon brush 230. Those skilled in the art may understand that the slip ring assembly 300 and the electrically connecting terminal 230 may also be arranged at other suitable positions, as long as it may be achieved that the slip ring assembly 300 and the electrically connecting terminal 230 may slide relative to each other, and that the slip ring assembly 300 may be electrically connected to the linear accelerator 200 by the carbon brush 230. The position of the slip ring assembly 300 and the position of the electrically connecting terminal 230 are not limited in the present application. For example, optionally, the slip ring assembly 300 may also be arranged on the accelerator stator 210, and the electrically connecting terminal 230 is arranged on the accelerator roller 220. When the linear accelerator 200 operates, the carbon brush 230 pivots together with the accelerator roller 220, and the slip ring assembly 300 is electrically connected to the linear accelerator 200 by the carbon brush 230. The bed assembly 240 is configured to be capable of at least partially moving into the hollow portion of the MRI device 100, and includes a bed board adapted to carry a patient 400 to be examined or to be treated. When the medical device 10 is in operation, the patient 400 is positioned on the bed board of the bed assembly 240, and the MRI device 100 is adapted to perform MRI on the region of interest (ROI) of the patient 400, to obtain magnetic resonance images. The linear accelerator 200 is adapted to perform a radiotherapy on the lesion tissue within the ROI based on the magnetic resonance images. Preferably, the MRI device 100 can perform the MRI on the tissue region of interest at any time, such as before, during, or after a radiotherapy. Preferably, the linear accelerator 200 can perform the radiotherapy on the lesion tissue within the ROI based on fusion images formed by the MRI images and images of other types, thereby further improving the accuracy of the radiotherapy.

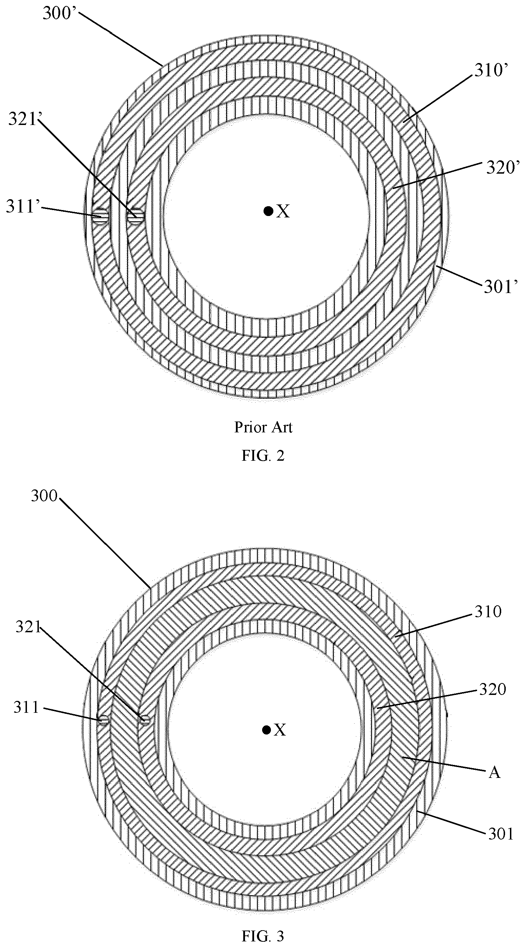

Referring to FIG. 2, FIG. 2 is a schematic plan view of the slip ring assembly 300' in the prior art. The slip ring assembly 300' includes: a base body 301', a first conductor 310', and a second conductor 320'. The first conductor 310' and the second conductor 320' are coaxially arranged on the same side of the base body 301', and a first brush 311' and a second brush 321' are arranged on one side of the first conductor 310' and on one side of the second conductor 320' respectively away from the base body 301'. When the slip ring assembly 300' is powered on, the magnetic fields generated by the first conductor 310' and the second conductor 320' will interact with the central magnetic field of the MRI device, which will interfere with a normal operation of the MRI device, thus reducing the accuracy of MRI.

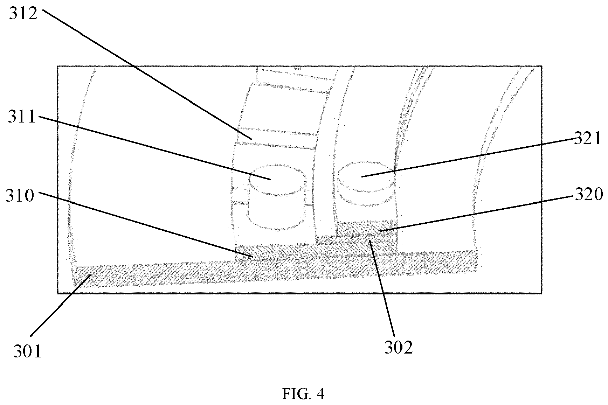

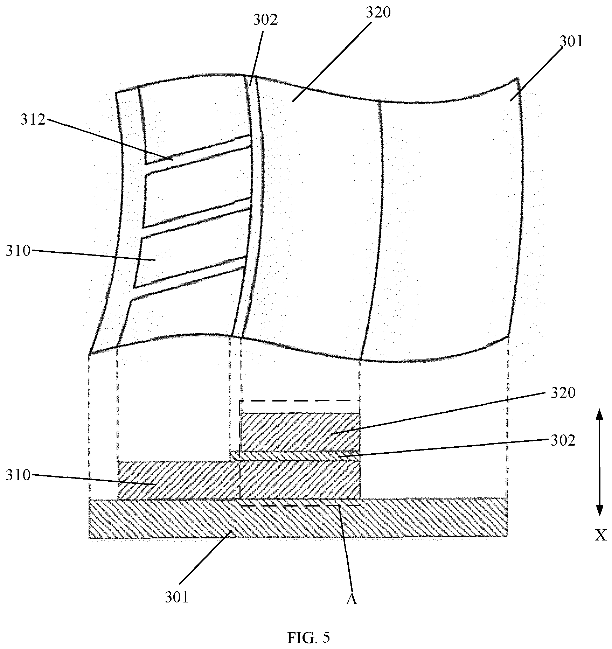

Referring to FIG. 1, FIG. 4 and FIG. 5, FIG. 4 is a perspective diagram of the slip ring assembly according to an embodiment of the present application, and FIG. 5 is a schematic sectional view of the slip ring assembly shown in FIG. 4. In the embodiment shown in FIG. 4 to FIG. 5, the slip ring assembly 300 includes: a base body 301, at least one first conductor 310, and at least one second conductor 320. The base body 301 is configured as an annular sheet structure and is adapted to be made of an insulating material. In the axial direction of the base body 301, the base body 301 includes a first side, and a second side opposite to the first side. The first side may be the side away from the accelerator roller 220 or the side facing the accelerator roller 220. The first conductor 310 is configured as an annular sheet structure, and the second conductor 320 is configured as an annular sheet structure. The first conductor 310 and the second conductor 320 are electrically insulated from each other. The projections of the first conductor 310 and second conductor 320 along the axial direction of the base body 301 (for example, the direction parallel to the X axis) at least partially overlap, that is, the projections have an overlapped area A.

In the embodiment shown in FIG. 4 to FIG. 5, the first conductor 310 and the second conductor 320 are arranged on the same side of the base body 301, such as on the first side or on the second side. Optionally, the first conductor 310 and the second conductor 320 may also be arranged in other manners, for example, the first conductor 310 and the second conductor 320 are arranged on different sides of the base body 301, which is not limited in this application, as long as it is satisfied that the projections of the first conductor 310 and second conductor 320 along the axial direction of the base body 301 at least partially overlap.

In the embodiment shown in FIG. 4 to FIG. 5, the base body 301, the first conductor 310 and the second conductor 320 are coaxially arranged. Optionally, the first conductor 310 and the second conductor 320 may also be arranged in other manners, for example, the base body 301, the first conductor 310, and the second conductor 320 are arranged non-axially, which is not limited in this application, as long as it is satisfied that the projections of the first conductor 310 and second conductor 320 along the axial direction of the base body 301 at least partially overlap.

In the embodiment shown in FIG. 4 and FIG. 5, the first conductor 310 and the second conductor 320 have the same inner diameter, and an outer diameter of the first conductor 310 is greater than an outer diameter of the second conductor 320. In this case, the overlapped area of the projections of the first conductor 310 and second conductor 320 along the axial direction is equal to the area of the projection of the second conductor 320 along the axial direction. Optionally, the first conductor 310 and the second conductor 320 may have the same inner diameter and the same outer diameter, or have different inner diameters and different outer diameters, or have the same inner diameter and different outer diameters, or have different inner diameters and the same outer diameter, as long as it is satisfied that when the side of the base body 301 where the first conductor 310 and the second conductor 320 are arranged is viewed along the axial direction of the base body 301, the first conductor 310 and the second conductor 320 may be seen at the same time, that is, the projections of the first conductor 310 and second conductor 320 at least partially overlap along the axial direction of the base body 3. For example, referring to FIG. 3, in the embodiment shown in FIG. 3, the first conductor 310 and the second conductor 320 may have different inner diameters and different outer diameters, and the projections of the first conductor 310 and second conductor 320 along the axial direction of the base body 301 have the overlapped area A.

In the slip ring assembly 300 according to this embodiment, the base body 301, the first conductor 310, and the second conductor 320 are all configured as the annular sheet structures, and the structure of the slip ring assembly 300 in the axial direction (namely an X-direction shown in FIG. 5) is very compact, so the slip ring assembly 300 requires less axial installation space. When the slip ring assembly 300 according to this embodiment is powered on, since the projections of the first conductor 310 and the second conductor 320 along the axial direction of the base body 301 at least partially overlap, the magnetic fields generated by the currents flowing through the portion of the first conductor 310 and the portion of the second conductor 320, which correspond to the overlapped area A respectively, at least partially offset each other, thereby reducing the interference magnetic field generated in the slip ring assembly 300, further reducing the interference in the central magnetic field of the MRI device, and optimizing the accuracy of the MRI.

Continuing to refer to FIG. 4 to FIG. 5, in this embodiment, a first insulator 302 is arranged between the first conductor 310 and the second conductor 320, so that the first conductor 310 and the second conductor 320 are electrically insulated from each other. Specifically, the first insulator 302 may be in an annular sheet shape, and the area of the projection of the first insulator 302 along the axial direction of the base body 301 is greater than or equal to the overlapped area of the first conductor 310 and the second conductor 320 along the axial direction.

Continuing to refer to FIG. 4 and FIG. 5, multiple first electrically isolating slots 312 are further formed in a portion of the first conductor 310, whose projection does not overlap the projection of the second conductor 320 in the axial direction of the base body 301. In the portion of the first conductor 310 where the first electrically isolating slot 312 is formed, the current cannot flow in a circumferential direction of the first conductor 310. In this embodiment, each first electrically isolating slot 312 is configured to extend linearly in the first conductor 310, and the plurality of first electrically isolating slots 312 are spaced apart from each other in the circumferential direction of the first conductor 310. Those skilled in the art should understand that the first electrically isolating slots 312 each may also have other shapes and arrangements, which are not limited in the application, as long as it may be satisfied that the current in the portion of the first conductor 310, where the first electrically isolating slots 312 are formed, cannot flow in the circumferential direction of the first conductor 310. Optionally, each of the first electrically isolating slots 312 may be configured to extend in the first conductor 310 in a curved or zigzag manner. Optionally, the plurality of first electrically isolating slots 312 are configured to extend from an outer edge of the portion of the first conductor 310 not overlapped by the second conductor 320, to an inner edge of the portion of the first conductor 310 not overlapped by the second conductor 320. Optionally, the plurality of first electrically isolating slots 312 are arranged at equal intervals in the circumferential direction of the first conductor 310. Optionally, the plurality of first electrically isolating slots 312 are configured to extend in the radial direction of the first conductor 310.

In this embodiment, the slip ring assembly 300 further includes at least one first brush 311 and at least one second brush 321. The first brush 311 is adapted to form an electrical contact with the first conductor 310. The second brush 321 is adapted to form an electrical contact with the second conductor 320. In this embodiment, the first brush 311 and the second brush 321 are arranged on the sides of the first conductor 310 and second conductor 320 respectively away from the base body 301. Those skilled in the art should understand that the first brush 311 and the second brush 321 may also be arranged at other suitable positions, as long as they can meet the requirements for forming the electrical contacts with the first conductor 310 and the second conductor 320 respectively. Optionally, the first brush 311 is disposed on a radial-direction inner surface or on a radial-direction outer surface of the first conductor 310. Optionally, the second brush 321 is disposed on a radial-direction inner surface or on a radial-direction outer surface of the second conductor 320.

Further, in another embodiment, an annular groove may also be formed on the first side of the base body 301, and the first conductor 310 is arranged in the annular groove. Preferably, a depth of the annular groove is equal to a thickness of the first conductor 310.

In the embodiment shown in FIG. 4 to FIG. 5, the number of the first conductor 310 and the number of second conductor 320 may be the same or different. The projection of each first conductor 310 along the axial direction of the base body 301 at least partially overlaps the projection of the second conductor 320 along the axial direction of the base body 301. A conductor group may include at least one first conductor 310 and at least one second conductor 320, and the slip ring assembly 300 includes at least one conductor group. Those skilled in the art should understand that the number of the first conductor 310 and the number of the second conductor 320 in each conductor group, and the number of the conductor group in the slip ring assembly 300 may be selected according to actual needs. For example, in an embodiment, the conductor group includes one first conductor 310 and one second conductor 320, and the slip ring assembly 300 includes one conductor group. For example, in another embodiment, the conductor group includes two first conductors 310 and one second conductor 320, and the slip ring assembly 300 includes two conductor groups.

In the slip ring assembly 300 according to this embodiment, the base body 301, the first conductor 310, and the second conductor 320 are all configured as the annular sheet structures, and the structure of the slip ring assembly 300 in the axial direction is very compact, so the slip ring assembly 300 requires less axial installation space. When the slip ring assembly 300 according to this embodiment is powered on, since the projections of the first conductor 310 and the second conductor 320 along the axial direction of the base body 301 at least partially overlap, the magnetic fields generated by the currents flowing through the portions of the first conductor 310 and the second conductor 320 corresponding to the overlapped area A respectively at least partially offset each other, thereby reducing the interference magnetic field generated in the slip ring assembly 300, thereby further reducing the interference in the central magnetic field of the MRI device. Furthermore, since the plurality of first electrically isolating slots 312 are formed in the first conductor 310, the current can only flow through the portions of the first conductor 310 and second conductor 320 corresponding to the overlapped area A respectively, thereby maximizing the reduction of the current-loop area of the slip ring assembly 300, further reducing the interference magnetic field generated in the slip ring assembly 300, and further reducing the interference in the central magnetic field of the MRI device, and ensuring the accuracy of MRI.

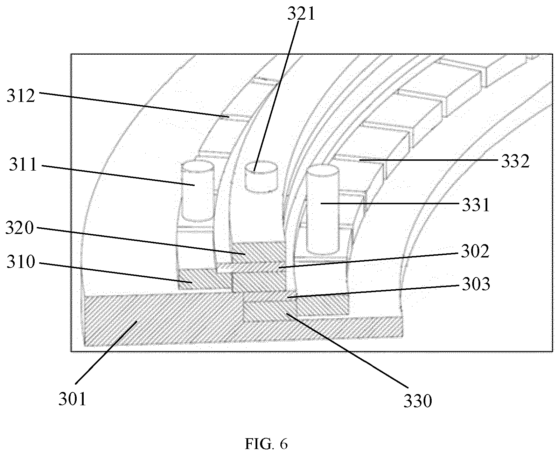

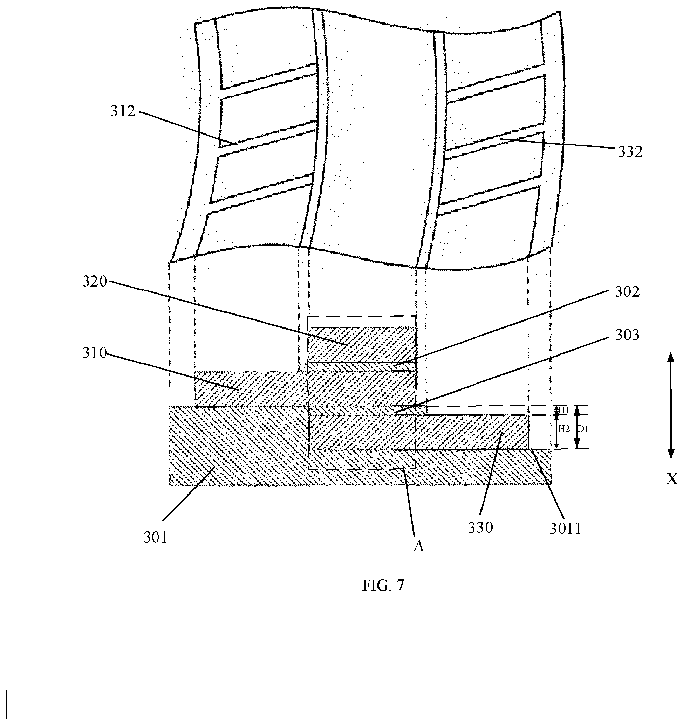

Referring to FIG. 1, 6, and 7, FIG. 6 is a perspective diagram of the slip ring assembly 300 according to an embodiment of the present application. FIG. 7 is a schematic sectional view of the slip ring assembly 300 shown in FIG. 6. In the embodiment shown in FIG. 6 and FIG. 7, the structural and positional relationships of the first conductor 310 and the second conductor 320 are similar to those of the first conductor 310 and the second conductor 320 in the embodiments shown in FIG. 3 to FIG. 5. In this embodiment, the annular assembly 300 further includes at least one third conductor 330. The third conductor 330 is configured as an annular sheet structure and is adapted to be fixed on the base body 301. The third conductor 330, the first conductor 310, and the second conductor 320 are arranged on the same side of the base body 301. The third conductor 330 is electrically insulated from the first conductor 310 or from the second conductor 320.

Optionally, the third conductor 330 is arranged between the first conductor 310 and the base body 301 along the axial direction of the base body 301, as shown in FIG. 7. A second insulator 303 is arranged between the third conductor 330 and the first conductor 310, and the second insulator 303 is adapted to electrically insulate the third conductor 330 from the first conductor 310. The third conductor 330 and the first conductor 310 at least partially overlap along the axial direction of the base body 301, such that the first conductor 310, the second conductor 320, and the third conductor 330 form an overlapped area A along the axial direction of the base body 301.

Optionally, the third conductor 330 is arranged on one side of the second conductor 320 along the axial direction of the base body 301, and a third insulator is arranged between the third conductor 330 and the second conductor 320. The third insulator is adapted to electrically insulate the third conductor 330 from the second conductor 320. The third conductor 330 and the second conductor 320 at least partially overlap along the axial direction of the base body 301, such that the first conductor 310, the second conductor 320, and the third conductor 330 form the overlapped area along the axial direction of the base body 301.

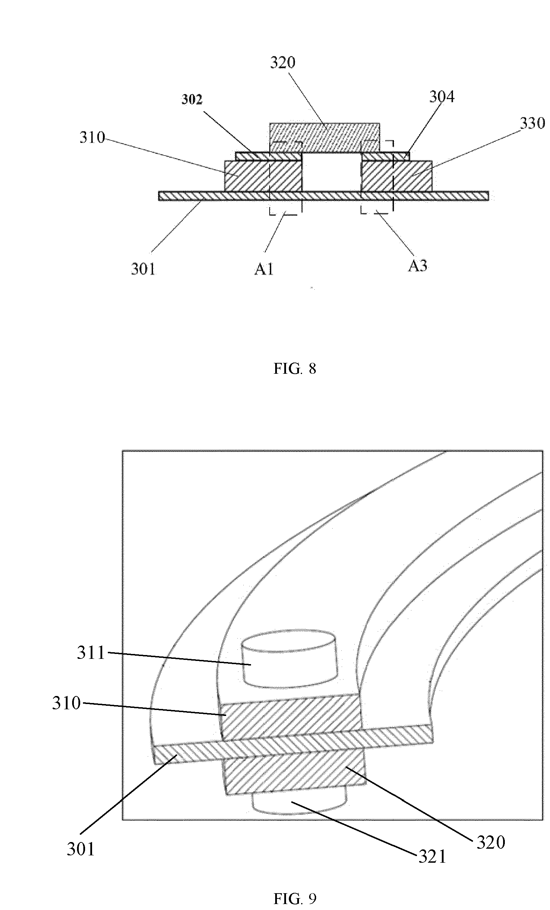

Optionally, the third conductor 330 is arranged between the second conductor 320 and the base body 301 along the axial direction of the base body 301. Referring to FIG. 8, a schematic sectional view of the slip ring assembly 330 according to an embodiment of the present application is shown. In the embodiment shown in FIG. 8, the first conductor 310 and the third conductor 330 are arranged on the same side of the base body 301, and the second conductor 320 is arranged on the same side of the first conductor 310 and third conductor 330 along the axial direction of the base body 301. The first insulator 302 is arranged between the first conductor 310 and the second conductor 320, so that the first conductor 310 and the second conductor 320 are electrically insulated from each other. A third insulator 304 is arranged between the third conductor 330 and the second conductor 320, so that the third conductor 330 and the second conductor 320 are electrically insulated from each other. The projections of the first conductor 310 and second conductor 320 along the axial direction of the base body 301 at least partially overlap to form an overlapped area A1, the projections of the third conductor 330 and second conductor 320 along the axial direction of the base body 301 at least partially overlap to form an overlapped area A3. Optionally, electrically isolating slots are formed in a portion of the first conductor 310, whose projection does not overlap the projection of the second conductor 320 in the axial direction of the base body 301. Optionally, electrically isolating slots are formed in a portion of the third conductor 330, whose projection does not overlap the projection of the second conductor 320 in the axial direction of the base body 301. Optionally, electrically isolating slots are formed in a portion of the second conductor 320, whose projection does not overlap the projections of the first conductor 310 and third conductor 330 in the axial direction of the base body 301.

In the embodiment shown in FIG. 6 and FIG. 7, an annular groove 3011 is further formed on the first side of the base body 301, and the third conductor 330 is arranged in the annular groove 3011. Optionally, the first conductor 310 is arranged in the annular groove 3011.

In the embodiment shown in FIG. 6 and FIG. 7, the second insulator 303 is configured as an annular sheet structure, and the area of the projection of the second insulator 303 along the axial direction of the base body 301 is greater than or equal to the overlapped area of the third conductor 330 and first conductor 310 along the axial direction. Optionally, a sum of a thickness H1 of the second insulator 303 and a thickness H2 of the third conductor 330 is equal to a depth D1 of the annular groove 3011.

In the embodiment that the first conductor 310 is arranged in the annular groove 3011, the third conductor 330 is arranged over the second conductor 320, and the second insulator 303 is arranged between the third conductor 330 and the second conductor 320. The second insulator 303 is adapted to electrically insulate the third conductor 330 from the second conductor 320. The third conductor 330 and the second conductor 310 at least partially overlap in the axial direction of the base body 301. Optionally, the sum of the thickness of the second insulator 303 and the thickness of the first conductor 310 is equal to the depth of the annular groove 3011.

In the slip ring assembly 300 of the embodiment shown in FIG. 6 and FIG. 7, the base body 301, the first conductor 310, the second conductor 320, and the third conductor 330 are all configured as the annular sheet structures, and the structure of the slip ring assembly 300 in the axial direction (namely an X-direction shown in FIG. 7) is very compact, so the slip ring assembly 300 requires less axial installation space. When the slip ring assembly 300 according to this embodiment is powered on, since the projections of the first conductor 310, second conductor 320 and third conductor 330 along the axial direction of the base body 301 at least partially overlap, the magnetic fields generated by the currents flowing through the portions of the first conductor 310, second conductor 320 and third conductor 330 corresponding to the overlapped area A respectively at least partially offset each other, thereby reducing the interference magnetic field generated in the slip ring assembly 300, thereby further reducing the interference in the central magnetic field of the MRI device, and optimizing the accuracy of the MRI.

Continuing to refer to FIG. 7, in this embodiment, a plurality of first electrically isolating slots 312 are formed in the portion of the first conductor 310 without being overlapped by the second conductor 320 in the axial direction of the base body 301, and a plurality of second electrically isolating slots 332 are formed in the portion of the third conductor 330 without being overlapped by the first conductor 310 in the axial direction of the base body 301. In the portions of the first conductor 310 and third conductor 330 where the isolation slots are formed, currents cannot flow in the circumferential directions of the first conductor 310 and third conductor 330 respectively. In this embodiment, each of the first electrically isolating slots 312 is configured to extend linearly in the first conductor 310, and each of the second electrically isolating slots 332 is configured to extend linearly in the third conductor 330. The plurality of first electrically isolating slots 312 are spaced apart from each other in the circumferential direction of the first conductor 310, and the plurality of third electrically isolating slots 332 are spaced apart from each other in the circumferential direction of the third conductor 310. Those skilled in the art should understand that the first electrically isolating slots 312 and the third electrically isolating slots 332 may have other shapes and arrangements, which are not limited in the application, as long as it may be satisfied that the currents in the portions of the first conductor 310 and third conductor 330, where the first electrically isolating slots 312 and the third electrically isolating slots 332 are formed respectively, cannot flow in the circumferential directions of the first conductor 310 and third conductor 330 respectively. Optionally, each of the first electrically isolating slots 312 may be configured to extend in the first conductor 310 in a curved or zigzag manner. Optionally, each of the third electrically isolating slots 332 may be configured to extend in the third conductor 330 in a curved or zigzag manner. Optionally, the plurality of first electrically isolating slots 312 are configured to extend from the outer edge of the portion of the first conductor 310, whose projection does not overlap the projection of the second conductor 320 in the axial direction of the base body 301, to the inner edge of the portion of the first conductor 310, whose projection does not overlap the projection of the second conductor 320 in the axial direction of the base body 301. Optionally, the plurality of third electrically isolating slots 332 are configured to extend from the outer edge of the portion of the third conductor 330, whose projection does not overlap the projection of the second conductor 320 in the axial direction of the base body 301 to the inner edge of the portion of the third conductor 330, whose projection does not overlap the projection of the second conductor 320 in the axial direction of the base body 301. Optionally, the plurality of first electrically isolating slots 312 are arranged at equal intervals in the circumferential direction of the first conductor 310. Optionally, the plurality of second electrically isolating slots 332 are arranged at equal intervals in the circumferential direction of the third conductor 330. Optionally, the plurality of first electrically isolating slots 312 each are configured to extend in the radial direction of the first conductor 310. Optionally, the plurality of third electrically isolating slots 332 each are configured to extend in the radial direction of the third conductor 330.

In the embodiment shown in FIG. 6 and FIG. 7, the slip ring assembly further includes a third brush 331 adapted to form an electrical contact with the third conductor 330. The third brush 331 is adapted to form the electrical contact with the third conductor 330. In this embodiment, the third brush 331 is arranged on a side of the third conductor 330 away from the base body 301. Those skilled in the art should understand that the third brush 331 may also be arranged at other suitable positions, as long as it may be satisfied that the third brush forms the electrical contact with the third conductor 330. Optionally, the third brush 331 is arranged on the radial-direction inner surface or on the radial-direction outer surface of the third conductor 330.

In the embodiment shown in FIG. 6 and FIG. 7, the numbers of the first conductor 310, second conductor 320 and third conductor 330 may be the same or different. At least one first conductor 310, at least one second conductor 320, and at least one third conductor 330 may form a conductor group, and the slip ring assembly 300 includes at least one conductor group. Those skilled in the art should understand that the numbers of the first conductor 310, second conductor 320 and third conductor 330 in each conductor group and the number of the conductor groups in the slip ring assembly 300 may be selected according to actual needs. For example, in an embodiment, the conductor group includes one first conductor 310, one second conductor 320, and one third conductor 330, and the slip ring assembly 300 includes one conductor group. In the radial direction of the base body 301, the diameters of the annular conductors in each annular conductor group gradually increase or decrease.

In the slip ring assembly 300 according to this embodiment, the base body 301, the first conductor 310, the second conductor 320, and the third conductor 330 are all configured as the annular sheet structures, and the structure of the slip ring assembly 300 in the axial direction is very compact, so the slip ring assembly 300 requires less axial installation space. When the slip ring assembly 300 according to this embodiment is powered on, since the projections of the first conductor 310, second conductor 320 and third conductor 330 along the axial direction of the base body 301 at least partially overlap, the magnetic fields generated by the current flowing through the portions of the first conductor 310, second conductor 320 and third conductor 330 corresponding to the overlapped area A respectively at least partially offset each other, thereby reducing the interference magnetic field generated in the slip ring assembly 300, and further reducing the interference in the central magnetic field of the MRI device. Furthermore, since the plurality of first electrically isolating slots 312 and the plurality of second electrically isolating slots 332 are formed in the first conductor 310 and the third conductor 330, respectively, the currents can only flow through the portions of the first conductor 310, second conductor 320 and third conductor 330 corresponding to the overlapped area A respectively, thereby maximizing the reduction of the current-loop area of the slip ring assembly 300, further reducing the interference magnetic field generated in the slip ring assembly 300, and thereby further reducing the interference in the central magnetic field of the MRI device, and ensuring the accuracy of the MRI.

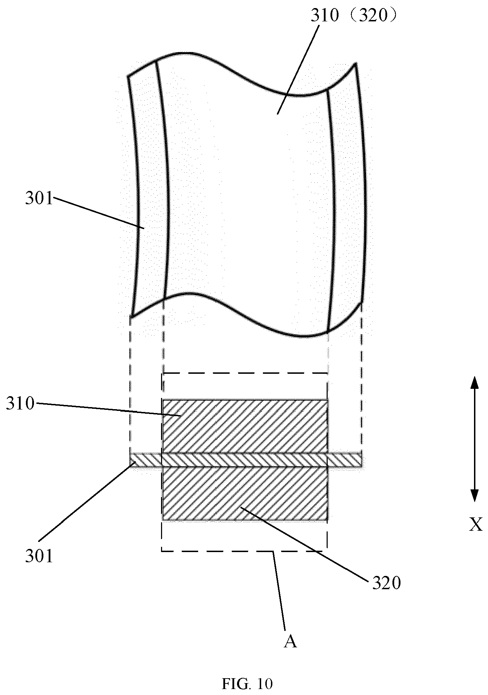

Referring to FIG. 9 and FIG. 10, FIG. 9 is a perspective diagram of the slip ring assembly according to the invention, and FIG. 10 is a schematic sectional view of the slip ring assembly shown in FIG. 9. In the embodiment shown in FIG. 9 and FIG. 10, the first conductor 310, the second conductor 320, the first brush 311 and the second brush 321 are similar to the embodiments shown in FIG. 3 to FIG. 5, and differ from the embodiments shown in FIG. 3 to FIG. 5 in that at least one first conductor 310 and at least one second conductor 320 are arranged on different sides of the base body 301 respectively.

In the embodiment shown in FIG. 9 and FIG. 10, the number of first conductor 310 and the number of the second conductor 320 may be the same or different. The conductor group includes at least one first conductor 310 and at least one second conductor 320, and the slip ring assembly 300 includes at least one conductor group. Those skilled in the art should understand that the numbers of the first conductor 310 and second conductor 320 in each conductor group and the number of conductor groups in the slip ring assembly 300 may be selected according to actual needs.

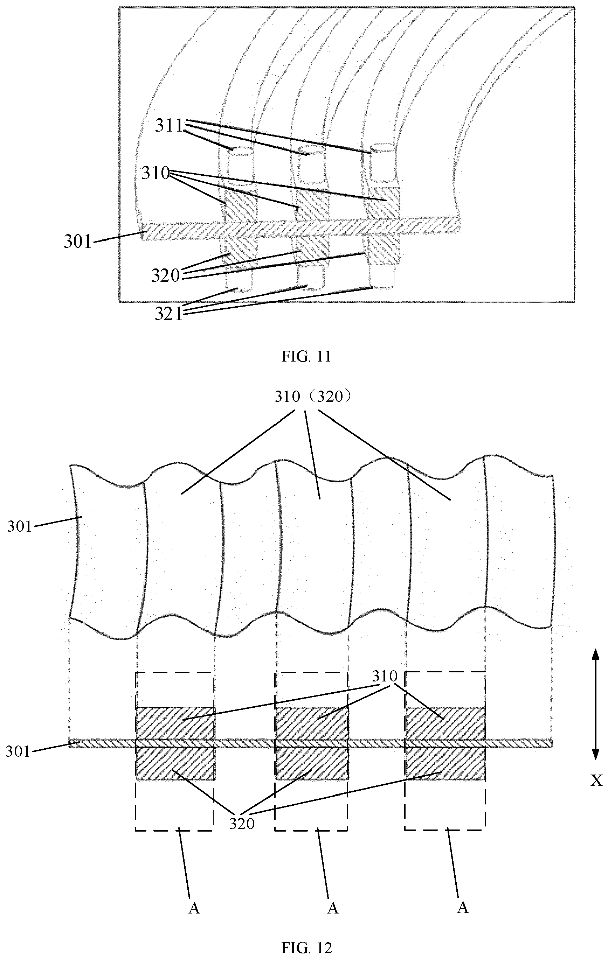

Optionally, in the embodiments shown in FIG. 11 and FIG. 12, the conductor group includes one first conductor 310 and one second conductor 320, and the slip ring assembly 300 includes three conductor groups. In each conductor group, the projections of the first conductor 310 and the second conductor 320 in the axial direction of the base body 301 at least partially overlap. Optionally, the slip ring assembly 300 may include another suitable number of conductor groups, such as one conductor group, two conductor groups, or four conductor groups, etc.

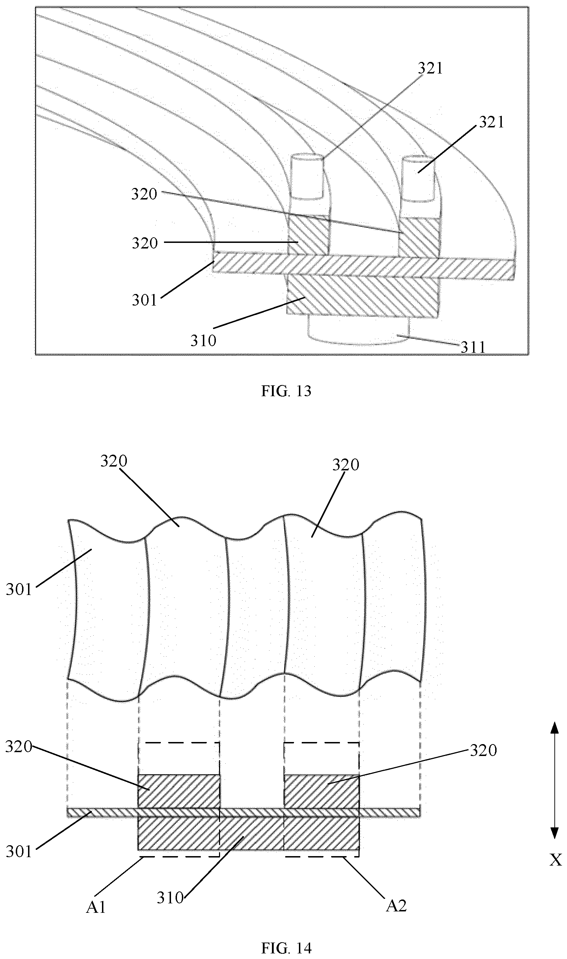

Optionally, in the embodiment shown in FIG. 13 and FIG. 14, the conductor group includes one first conductor 310 and two second conductors 320, and the slip ring assembly 300 includes one conductor group. In the conductor group, the projection of the first conductor 310 at least partially overlaps the projections of the two second conductors 320 in the axial direction of the base body 301, respectively. Optionally, the slip ring assembly 300 may include another suitable number of conductor groups, such as two conductor groups, three conductor groups, or four conductor groups, etc.

In the embodiments in FIG. 9 and FIG. 10, FIG. 11 and FIG. 12, and FIG. 13 and FIG. 14, the base body 301, the first conductor 310 and the second conductor 320 of the slip ring assembly 300 are all configured as the annular sheet structures, and the structure of the slip ring assembly 300 in the axial direction of the base body 301 (namely an X-direction shown in FIGS. 10, 12 and 14) is very compact, so the slip ring assembly 300 requires less axial installation space. When the slip ring assembly 300 according to this embodiment is powered on, since the projections of the first conductor 310 and second conductor 320 along the axial direction of the base body 301 at least partially overlap, the magnetic fields generated by the current flowing through the portions of the projections of the first conductor 310 and second conductor 320 corresponding to the overlapped area A respectively at least partially offset each other, thereby reducing the interference magnetic field generated in the slip ring assembly 300, further reducing the interference in the central magnetic field of the MRI device, and optimizing the accuracy of the MRI.

Further, in the embodiment shown in FIG. 13 and FIG. 14, a plurality of third electrically isolating slots (not shown) are formed in the portion of the first conductor 310 without being overlapped by the second conductor 320 in the axial direction of the base body 301, and the current cannot flow in the circumferential direction through the portion of the first conductor 310, where the third electrically isolating slots are formed. In this embodiment, each of the third electrically isolating slots is configured to extend linearly in the first conductor 310, and the plurality of third electrically isolating slots are spaced apart from each other in the circumferential direction of the first conductor 310. Those skilled in the art should understand that the third electrically isolating slot may also have other shapes and arrangements, which are not limited in the application, as long as it may be satisfied that the current cannot flow in the circumferential direction of the first conductor 310 through the portion of the first conductor 310, where the third electrically isolating slots are formed. Optionally, each third electrically isolating slot is configured to extend in the first conductor 310 in a curved or zigzag manner. Optionally, the plurality of third electrically isolating slots are configured to extend from the outer edge of the portion of the first conductor 310 without being overlapped by the second conductor 320 to the inner edge of the portion of the first conductor 310 without being overlapped by the second conductor 320. Optionally, the plurality of third electrically isolating slots are arranged at equal intervals in the circumferential direction of the first conductor 310. Optionally, the plurality of third electrically isolating slots are configured to extend in the radial direction of the first conductor 310.

Further, in the embodiment shown in FIG. 13 and FIG. 14, the slip ring assembly 330 includes at least two second conductors 320. The two second conductors 320 are arranged on the same side of the base body 301, and the at least two second conductors 320 are electrically insulated from each other. The projection of the first conductor 310 partially overlaps the projections of the two second conductors 320 along the axial direction of the base body 301, respectively, to form a first overlapped area A1 and a second overlapped area A2. Optionally, a gap is formed between the two second conductors 320, so that the at least two second conductors 320 are electrically insulated from each other. Further, insulating material is provided in the gap between the two second conductors 320, so that the at least two second conductors 320 are electrically insulated from each other.

In the slip ring assembly 300 according to this embodiment, the base body 301, the first conductor 310, and the second conductor 320 are all configured as the annular sheet structures, and the structure of the slip ring assembly 300 in the axial direction is very compact, so the slip ring assembly 300 requires less installation space. When the slip ring assembly 300 according to this embodiment is powered on, since the projections of the first conductor 310 and the second conductor 320 along the axial direction of the base body 301 at least partially overlap, the magnetic fields generated by the currents flowing through the portions of the first conductor 310 and the second conductor 320 corresponding to the overlapped area A respectively at least partially offset each other, thereby reducing the interference magnetic field generated in the slip ring assembly 300, further reducing the interference in the central magnetic field of the MRI device. Furthermore, since the plurality of first electrically isolating slots 312 are formed in the first conductor 310, the currents can only flow through the portions of the first conductor 310 and second conductor 320 corresponding to the overlapped area A1 or A2 respectively, thereby maximizing the reduction of the current-loop area of the slip ring assembly 300, further reducing the interference magnetic field generated in the slip ring assembly 300, further reducing the interference in the central magnetic field of the MRI device, and ensuring the accuracy of the MRI.

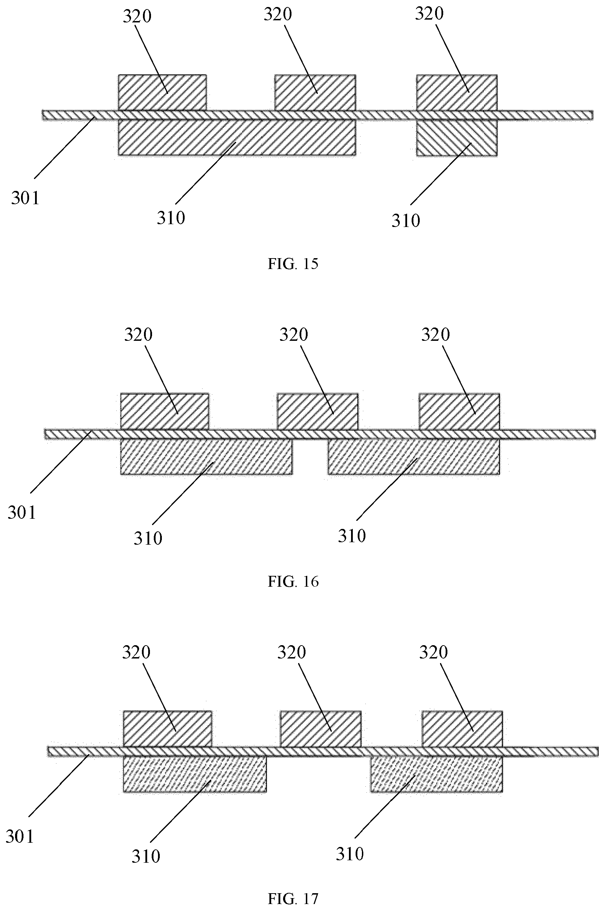

Referring to FIG. 15, FIG. 15 shows a conductor group of the slip ring assembly 300 according to an embodiment of the present invention. The conductor group includes at least two first conductors 310 and at least three second conductors 320. The first conductor 310 and the second conductor 320 are respectively arranged on different sides of the base body 301 in the axial direction. The projections of one first conductor 310 and two of the second conductors 320 along the axial direction of the base body 301 at least partially overlap to obtain an overlapped area. The projection of another first conductor 310 and another second conductor 320 along the axial direction of the base body 301 at least partially overlaps to obtain another overlapped area. In this embodiment, the slip ring assembly 300 includes at least one conductor group. Optionally, the slip ring assembly 300 may include another suitable number of conductor groups, such as two conductor groups, three conductor groups, or four conductor groups, etc.

Referring to FIG. 16, FIG. 16 shows a schematic sectional view of the slip ring assembly 300 according to an embodiment of the present application. The conductor group includes at least two first conductors 310 and at least three second conductors 320. The first conductor 310 and the second conductor 320 are arranged on different sides of the base body 301 in the axial direction respectively. The projection of each first conductor 310 and the projections of two of the second conductors 320 along the axial direction of the base body 301 at least partially overlap to obtain an overlapped area. In this embodiment, the slip ring assembly 300 includes at least one conductor group. Optionally, the slip ring assembly 300 may include another suitable number of conductor groups, such as two conductor groups, three conductor groups, or four conductor groups, etc.

Referring to FIG. 17, FIG. 17 shows the slip ring assembly 300 according to an embodiment of the present application. The conductor group includes at least two first conductors 310 and at least three second conductors 320. The first conductor 310 and the second conductor 320 are arranged on different sides of the base body 301 in the axial direction respectively. The projection of at least one first conductor 310 and the projection of the at least one second conductor 320 along the axial direction of the base body 301 at least partially overlap to obtain an overlapped area, and the projection of at least one second conductor 320 and the projection of any one first conductor 310 in the axial direction of base body 301 do not overlap. In this embodiment, the slip ring assembly 300 includes at least one conductor group. Optionally, the slip ring assembly 300 may include another suitable number of conductor groups, such as two conductor groups, three conductor groups, or four conductor groups, etc.

In the embodiments shown in FIG. 15 to FIG. 17, optionally, electrically isolating slots are formed in the portion of the first conductor 310, whose projection does not overlap the projection of the second conductor 320 along the axial direction of the base body 301. Optionally, electrically isolating slots are formed in the portion of the second conductor 320, whose projection does not overlap the projection of the first conductor 310 in the axial direction of the base body 301.

In the embodiments shown in FIG. 15 to FIG. 17, the base body 301, the first conductor 310, and the second conductor 320 are all configured as the annular sheet structures, and the structure of the slip ring assembly 300 in the axial direction is very compact, so the slip ring assembly 300 requires less installation space. When the slip ring assembly 300 according to this embodiment is powered on, since the projections of the first conductor 310 and second conductor 320 along the axial direction of the base body 301 at least partially overlap, the magnetic fields generated by the currents flowing through the portions of the first conductor 310 and second conductor 320 corresponding to the overlapped area A respectively at least partially offset each other, thereby reducing the interference magnetic field generated in the slip ring assembly 300, further reducing the interference in the central magnetic field of the MRI device. Furthermore, since the plurality of electrically isolating slots are formed in the first conductor 310 or in the second conductor 320, the currents can only flow through the portions of the first conductor 310 and second conductor 320 corresponding to the overlapped area respectively, thereby maximizing the reduction of the current-loop area of the slip ring assembly 300, further reducing the interference magnetic field generated in the slip ring assembly 300, further reducing the interference in the central magnetic field of the MRI device, and ensuring the accuracy of the MRI.

The embodiments above only represent several implementation modes of the present application, and the description thereof is relatively specific and detailed, but it should not be construed as limiting the scope of the patent.

## Claims

1. A slip ring assembly (300), used in a magnetic field of a magnetic resonance imaging device (100), and comprising:
a base body (301), the base body (301) having an annular shape and made of an insulating material, and the base body (301) comprising a first outer side and a second outer side opposite to the first outer side along an axial direction of the base body (301);
at least one first conductor (310), the first conductor (310) having an annular shape and being fixed on the first outer side; and
at least one second conductor (320), the second conductor (320) having an annular shape;
wherein the first conductor (310) and the second conductor (320) are electrically insulated from each other, and a projection of the first conductor (310) and a projection of the second conductor (320) along the axial direction of the base body (301) at least partially overlap,
**characterized in that** the at least one second conductor (320) is fixed on the second outer side.

2. The slip ring assembly (300) according to claim 1, wherein an annular groove (3011) is formed on the base body (301);
optionally, the first conductor (310) is disposed in the annular groove (3011).

3. The slip ring assembly (300) according to claim 1, wherein a plurality of first electrically isolating slots (312) are formed in a portion of the first conductor (310), whose projection does not overlap the projection of the second conductor (320) along the axial direction of the base body (301); and the plurality of first electrically isolating slots (312) are spaced apart from each other along a circumferential direction of the first conductor (310).

4. The slip ring assembly (300) according to claim 3, wherein the plurality of first electrically isolating slots (312) are arranged at equal intervals in the circumferential direction of the first conductor (310); or
each of the plurality of first electrically isolating slots (312) is configured to extend in a radial direction of the first conductor (310).

5. The slip ring assembly (300) according to claim 1, wherein a conductor group comprises at least one first conductor (310) and at least one second conductor (320); and the slip ring assembly (300) comprises at least one conductor group.

6. The slip ring assembly (300) according to claim 5, wherein each conductor group comprises one first conductor (310) and one second conductor (320), and the first conductor (310) and the second conductor (320) are configured that the projection of the first conductor (310) and the projection of the second conductor (320) along the axial direction of the base body (301) completely overlap.

7. The slip ring assembly (300) according to claim 5, wherein each conductor group comprises one first conductor (310) and at least two second conductors (320); the projection of the first conductor (310) at least partially overlaps projections of the at least two second conductors (320) in the axial direction of the base body (301), respectively; and the at least two second conductors (320) are configured to be electrically insulated from each other.

8. The slip ring assembly (300) according to claim 1, wherein the number of the first conductor (310) is different from the number of the second conductor (320); or
a projection of each first conductor (310) along the axial direction of the base body (301) at least partially overlaps a projection of one second conductor (320) along the axial direction of the base body (301).

9. The slip ring assembly (300) according to claim 1, further comprising a first brush (311) and a second brush (321), wherein the first brush (311) is adapted to form an electrical contact with the first conductor (310), and the second brush (321) is adapted to form an electrical contact with the second conductor (320).

10. A medical device, comprising a treatment device (200), **characterized in that** the treatment device (200) comprises the slip ring assembly (300) according to any one of claims 1 to 9, and the slip ring assembly (300) is adapted to act as a power supply medium or a communication medium of the treatment device (200);
optionally, the medical device further comprises a magnetic resonance imaging device (100).

## Patentansprüche

1. Eine Schleifringanordnung (300), die in einem Magnetfeld einer Magnetresonanzbildgebungsvorrichtung (100) verwendet wird und Folgendes beinhaltet:
einen Grundkörper (301), wobei der Grundkörper (301) eine Ringform aufweist und aus einem isolierenden Material hergestellt ist und der Grundkörper (301) eine erste Außenseite und eine zweite Außenseite gegenüber der ersten Außenseite entlang einer axialen Richtung des Grundkörpers (301) beinhaltet;
mindestens einen ersten Leiter (310), wobei der erste Leiter (310) eine Ringform aufweist und an der ersten Außenseite befestigt ist; und
mindestens einen zweiten Leiter (320), wobei der zweite Leiter (320) eine Ringform aufweist;
wobei der erste Leiter (310) und der zweite Leiter (320) elektrisch voneinander isoliert sind und sich eine Projektion des ersten Leiters (310) und eine Projektion des zweiten Leiters (320) entlang der axialen Richtung des Grundkörpers (301) mindestens teilweise überlappen,
**dadurch gekennzeichnet, dass** der mindestens eine zweite Leiter (320) an der zweiten Außenseite befestigt ist.

2. Schleifringanordnung (300) gemäß Anspruch 1, wobei eine Ringnut (3011) an dem Grundkörper (301) ausgebildet ist;
wobei der erste Leiter (310) optional in der Ringnut (3011) angeordnet ist.

3. Schleifringanordnung (300) gemäß Anspruch 1, wobei eine Vielzahl von ersten elektrisch isolierenden Schlitzen (312) in einem Abschnitt des ersten Leiters (310) ausgebildet ist, dessen Projektion sich nicht mit der Projektion des zweiten Leiters (320) entlang der axialen Richtung des Grundkörpers (301) überlappt; und die Vielzahl von ersten elektrisch isolierenden Schlitzen (312) entlang einer Umfangsrichtung des ersten Leiters (310) voneinander beabstandet ist.

4. Schleifringanordnung (300) gemäß Anspruch 3, wobei die Vielzahl von ersten elektrisch isolierenden Schlitzen (312) in gleichen Abständen in der Umfangsrichtung des ersten Leiters (310) angeordnet ist; oder
jeder der Vielzahl von ersten elektrisch isolierenden Schlitzen (312) konfiguriert ist, um sich in einer radialen Richtung des ersten Leiters (310) zu erstrecken.

5. Schleifringanordnung (300) gemäß Anspruch 1, wobei eine Leitergruppe mindestens einen ersten Leiter (310) und mindestens einen zweiten Leiter (320) beinhaltet; und die Schleifringanordnung (300) mindestens eine Leitergruppe beinhaltet.

6. Schleifringanordnung (300) gemäß Anspruch 5, wobei jede Leitergruppe einen ersten Leiter (310) und einen zweiten Leiter (320) beinhaltet und der erste Leiter (310) und der zweite Leiter (320) konfiguriert sind, sodass sich die Projektion des ersten Leiters (310) und die Projektion des zweiten Leiters (320) entlang der axialen Richtung des Grundkörpers (301) vollständig überlappen.

7. Schleifringanordnung (300) gemäß Anspruch 5, wobei jede Leitergruppe einen ersten Leiter (310) und mindestens zwei zweite Leiter (320) beinhaltet; die Projektion des ersten Leiters (310) sich jeweils mindestens teilweise mit Projektionen der mindestens zwei zweiten Leiter (320) in der axialen Richtung des Grundkörpers (301) überlappt; und die mindestens zwei zweiten Leiter (320) konfiguriert sind, um elektrisch voneinander isoliert zu sein.

8. Schleifringanordnung (300) gemäß Anspruch 1, wobei sich die Anzahl der ersten Leiter (310) von der Anzahl der zweiten Leiter (320) unterscheidet; oder
sich eine Projektion jedes ersten Leiters (310) entlang der axialen Richtung des Grundkörpers (301) mindestens teilweise mit einer Projektion eines zweiten Leiters (320) entlang der axialen Richtung des Grundkörpers (301) überlappt.

9. Schleifringanordnung (300) gemäß Anspruch 1, die ferner eine erste Bürste (311) und eine zweite Bürste (321) beinhaltet, wobei die erste Bürste (311) eingerichtet ist, um einen elektrischen Kontakt mit dem ersten Leiter (310) herzustellen, und die zweite Bürste (321) eingerichtet ist, um einen elektrischen Kontakt mit dem zweiten Leiter (320) herzustellen.

10. Eine medizinische Vorrichtung, die eine Behandlungsvorrichtung (200) beinhaltet,
**dadurch gekennzeichnet, dass** die Behandlungsvorrichtung (200) die Schleifringanordnung (300) gemäß einem der Ansprüche 1 bis 9 beinhaltet und die Schleifringanordnung (300) eingerichtet ist, um als ein Stromversorgungsmedium oder ein Kommunikationsmedium der Behandlungsvorrichtung (200) zu wirken;
optional beinhaltet die medizinische Vorrichtung ferner eine Magnetresonanzbildgebungsvorrichtung (100).

## Revendications

1. Un ensemble bague collectrice (300), utilisé dans un champ magnétique d'un dispositif d'imagerie par résonance magnétique (100), et comprenant :
un corps formant base (301), le corps formant base (301) présentant une forme annulaire et étant réalisé en un matériau isolant, et le corps formant base (301) comprenant un premier côté extérieur et un deuxième côté extérieur opposé au premier côté extérieur le long d'une direction axiale du corps formant base (301) ;
au moins un premier conducteur (310), le premier conducteur (310) présentant une forme annulaire et étant fixé sur le premier côté extérieur ; et
au moins un deuxième conducteur (320), le deuxième conducteur (320) présentant une forme annulaire ;
où le premier conducteur (310) et le deuxième conducteur (320) sont isolés électriquement l'un de l'autre, et une projection du premier conducteur (310) et une projection du deuxième conducteur (320) le long de la direction axiale du corps formant base (301) se chevauchent au moins partiellement,
**caractérisé en ce que** l'au moins un deuxième conducteur (320) est fixé sur le deuxième côté extérieur.

2. L'ensemble bague collectrice (300) selon la revendication 1, où une rainure annulaire (3011) est formée sur le corps formant base (301) ;
facultativement, le premier conducteur (310) est disposé dans la rainure annulaire (3011).

3. L'ensemble bague collectrice (300) selon la revendication 1, où une pluralité de premières fentes électriquement isolantes (312) sont formées dans une portion du premier conducteur (310), dont la projection ne chevauche pas la projection du deuxième conducteur (320) le long de la direction axiale du corps formant base (301) ; et la pluralité de premières fentes électriquement isolantes (312) sont espacées les unes des autres le long d'une direction circonférentielle du premier conducteur (310).

4. L'ensemble bague collectrice (300) selon la revendication 3, où la pluralité de premières fentes électriquement isolantes (312) sont agencées à intervalles égaux dans la direction circonférentielle du premier conducteur (310) ; ou
chaque fente de la pluralité de premières fentes électriquement isolantes (312) est configurée pour s'étendre dans une direction radiale du premier conducteur (310).

5. L'ensemble bague collectrice (300) selon la revendication 1, où un groupe de conducteurs comprend au moins un premier conducteur (310) et au moins un deuxième conducteur (320) ; et l'ensemble bague collectrice (300) comprend au moins un groupe de conducteurs.

6. L'ensemble bague collectrice (300) selon la revendication 5, où chaque groupe de conducteurs comprend un premier conducteur (310) et un deuxième conducteur (320), et le premier conducteur (310) et le deuxième conducteur (320) sont configurés de sorte que la projection du premier conducteur (310) et la projection du deuxième conducteur (320) le long de la direction axiale du corps formant base (301) se chevauchent complètement.

7. L'ensemble bague collectrice (300) selon la revendication 5, où chaque groupe de conducteurs comprend un premier conducteur (310) et au moins deux deuxièmes conducteurs (320) ; la projection du premier conducteur (310) chevauche au moins partiellement des projections des au moins deux deuxièmes conducteurs (320) dans la direction axiale du corps formant base (301), respectivement ; et les au moins deux deuxièmes conducteurs (320) sont configurés pour être isolés électriquement les uns des autres.

8. L'ensemble bague collectrice (300) selon la revendication 1, où le nombre de premiers conducteurs (310) est différent du nombre de deuxièmes conducteurs (320) ; ou
une projection de chaque premier conducteur (310) le long de la direction axiale du corps formant base (301) chevauche au moins partiellement une projection d'un deuxième conducteur (320) le long de la direction axiale du corps formant base (301).

9. L'ensemble bague collectrice (300) selon la revendication 1, comprenant en outre un premier balai (311) et un deuxième balai (321), où le premier balai (311) est conçu pour former un contact électrique avec le premier conducteur (310), et le deuxième balai (321) est conçu pour former un contact électrique avec le deuxième conducteur (320).

10. Un dispositif médical, comprenant un dispositif de traitement (200), **caractérisé en ce que** le dispositif de traitement (200) comprend l'ensemble bague collectrice (300) selon l'une quelconque des revendications 1 à 9, et l'ensemble bague collectrice (300) est conçu pour agir comme un moyen d'alimentation électrique ou un moyen de communication du dispositif de traitement (200) ;
facultativement, le dispositif médical comprend en outre un dispositif d'imagerie par résonance magnétique (100).
